# EUROPEAN PATENT SPECIFICATION

(11) **EP 0 701 835 B1**
(45) Date of publication and mention of the grant of the patent: **12.11.2003**
(21) Application number: 95202503.9
(22) Date of filing: 15.09.1995
(51) Int. Cl.: A61M 25/01

(54) **MR-visible catheter comprising hardened magnetisable material**
NMR-sichtbarer Katheter mit ausgehärtetem, magnetisierbarem Material
Cathéther visible par imagerie à résonance magnétique avec matériau magnétisable durci

(30) Priority: 19.09.1994 NL 9401518
(43) Date of publication of application: 20.03.1996
(73) Proprietor: CORDIS EUROPA N.V., NL-9301 LJ Roden (NL)
(72) Inventor: Weber, Jan, NL-9302 ER Roden (NL); Van Erp, Wilhelmus P. M. M., NL-9351 KS Leek (NL)
(74) Representative: 't Jong, Bastiaan Jacob

(56) References cited:
- WO-A-85/04330
- US-A- 5 154 179

## Description

The invention relates to a catheter designed for treatment or investigation of a patient while the area to be treated or investigated is visualised by means of NMR techniques.

Known catheters are manufactured in such a way that they become visible when subjected to X-rays, so that the catheter or at least sections thereof can be made visible on an X-ray screen during treatment or investigation of a patient.

The usual catheters are however not or badly visible when using an NMR device.

The American patent specification 5 154 179, which is considered to represent the closest prior art, describes a catheter in which ferromagnetic material has been received in the material of which the catheter has been made or which is used in conjunction with a contrast medium containing paramagnetic material. The drawback of using ferromagnetic material is that the image created by the catheter on the NMR screen outshines the surrounding, so that the latter can not be seen clearly. In order to use a contrast medium containing paramagnetic material a separate channel in the catheter is required, as a consequence of which the diameter will remain comparatively large. Especially in the case of neurological applications, when the use of NMR techniques is particularly desirable, catheters should have very small diameters.

The object of the invention is to provide a catheter with a very small diameter which can nevertheless be rendered clearly visible under NMR circumstances.

This aim is achieved with the catheter as characterised in claim 1. By receiving the particulate magnetic material in a material which will harden, such as a glue or a paint, an accurately measured quantity of magnetic material can be arranged at required positions. Thus it is for example possible to keep sections of the catheter, which during use will be situated in areas which are to be studied carefully, free of magnetic material, so that there will be no disturbance of the image at that site. An additional advantage is that it is possible to carefully measure off the quantity of particulate magnetic material to be used.

A very favourable embodiment is characterized in claim 2. In this way the visibility of the catheter is independent of the direction of the magnetic field.

A suitable embodiment in this regard is characterized in claim 3. The hardened adhesive medium comprising the particulate magnetic material forms, so to speak, a layer integrated in the catheter, which can be made visible on the screen. As the adhesive medium is enclosed both by the material of which the catheter has been made and the end-section, there can be no contact with the body or body fluids of the patient, or fluids present inside the lumen or flowing through it. Material which as such is harmful to man, can therefore also be used by way of particulate magnetic material. Some paramagnetic materials which are particularly suitable for application according to the invention but are harmful to man, can therefore be used nevertheless.

The measure of claim 4 also provides for a visibility of the catheter irregardless of the direction of the magnetic field.

If the catheter is manufactured in accordance with claim 5, any section of the catheter can, as mentioned before, be made visible to any required degree.

With this application it also may be desirable to use material which, as such, is harmful to man. In that case the measure as set out in claim 6 is preferably employed. The metallic paint, together with a coating, can be applied very thinly, so that the effective diameter of the catheter is not affected to any significant, unfavourable degree.

Claim 7 and 8 list suitable materials for application with the catheter according to the invention.

The invention will be explained in the following description with reference to the attached drawings.

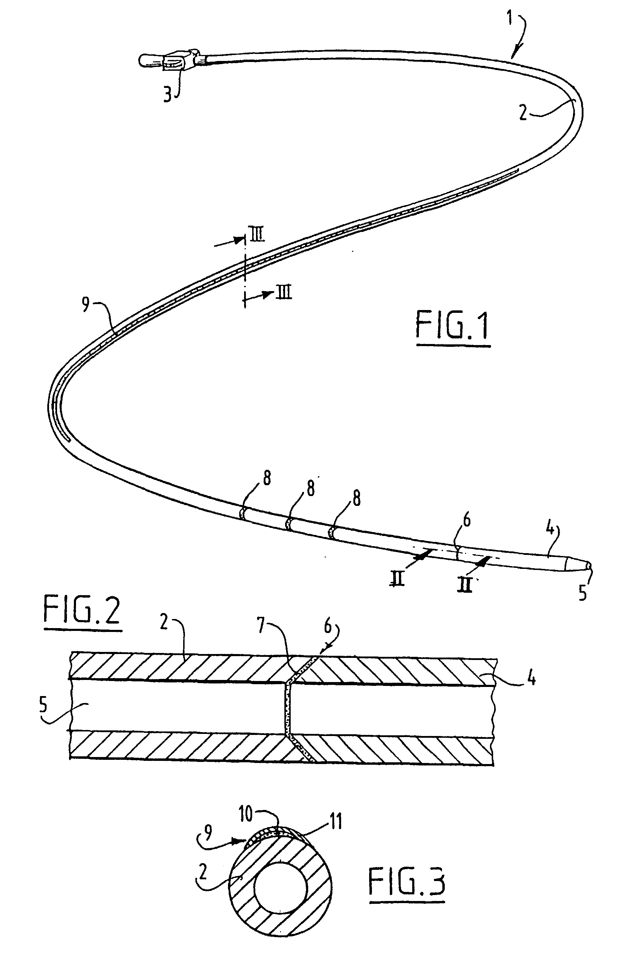

Figure 1 shows a perspective view of a catheter according to a preferred embodiment of the invention; figure 2 illustrates a cross-section taken along the line II-II of figure 1, and figure 3 represents a cross-sectional view taken along the line III-III of figure 1.

The catheter 1 shown in general in figure 1, comprises a basic body 2 with a Luer-type fitting 3 at the proximal end. At the distal end the catheter 1 comprises an end-section 4 which, at a connecting surface 6, is connected to the basic body 2. The end-section 4 has been made of a more flexible type of material than the basic body 2, so that the end-section is a-traumatic.

Inside the catheter 1, a lumen 5 extends from the connecting member 3 to the distal tip.

In order to make the catheter 1 properly visible when using NMR techniques, it has been provided with a number of marking-bands 8 and a longitudinal stripe 9. These markers are formed by a layer of hardened material comprising particles of magnetic material. In the magnetic field of an NMR device these particles will disturb the uniformity of the magnetic field, which will become visible on the screen of the device.

At surface 6, the end-section 4 is connected by means of an adhesive medium 7 to the basic body 2. This adhesive medium also comprises particulate magnetic material, so that the position of the connecting surface 6 will be made visible on the NMR screen.

The particulate material received in the adhesive medium 7 can be paramagnetic material which, as such is harmful to man. As it will be enclosed however, it can be employed without causing harmful effects.

The choice of the magnetic material depends amongst other things on the circumstances under which the catheter is to be used. A ferromagnetic or strong paramagnetic material will already give a very clear picture when used in very small quantities. Weaker paramagnetic materials are to be used in comparatively stronger concentrations in order to obtain the same image formation.

As is shown in figure 3, the stripe 9 of catheter 1 is made up of two layers. The bottom layer 10 comprises the hardened material with the particulate magnetic material. With this embodiment a magnetic material has been chosen which as such is harmful to man. A coating 11 has been applied to the inner layer 10, which thus prevents direct contact between the harmful material and the body or body fluids of the patient.

As the material can be applied in liquid state and hardens subsequently, the layer can be very thin, so that the overall thickness of the catheter 1 can remain limited, which, especially in the case of neurological applications, is very important.

## Claims

1. Catheter (1) comprising particulate magnetic material (8,9,10) a tube-like basic body (2) with a distal and a proximal end and at least one lumen, **characterized in that** the particulate magnetic matenal (8,9,10) has been received in material hardened by curing, such as glue or paint, connected to the basic body (2).

2. Catheter as claimed in claim 1, wherein the hardened material is provided in the form of at least one ring.

3. Catheter as claimed in claim 2, wherein the catheter comprises an end-section connected to the basic body at the distal end, and wherein the hardened material consists of an adhesive medium applied between the basic body and the end-section.

4. Catheter as claimed in claim 1, wherein the hardened material is provided in the form of at least one helical band.

5. Catheter as claimed in claim 1, wherein the hardened material comprising the magnetic material consists of a metallic paint applied to the outer surface of the catheter.

6. Catheter as claimed in claim 5, wherein the magnetic material is harmful to man, and a coating of material free of magnetic material has been applied on top of the hardened material.

7. Catheter as claimed in one of the previous claims, wherein the particulate magnetic material consists of ferromagnetic material.

8. Catheter as claimed in one of the previous claims, wherein the particulate magnetic material consists of strong paramagnetic material of the group comprising copper, manganese, chromium, nickel, gadolinium and dysprosium and mixtures, alloys and salts thereof.

9. Method of making a MR-visible catheter, by providing a catheter (1) and particulate magnetic material (8,9,10), **characterized in that** the particulate magnetic material has been received in material hardened by curing, such as glue or paint, connected to the basic body (2) of the catheter (1).

## Patentansprüche

1. Katheter (1) umfassend partikuläres magnetisches Material (8, 9, 10), einen rohrförmigen Grundkörper (2) mit einem distalen und einem proximalen Ende und wenigstens ein Lumen, **dadurch gekennzeichnet, daß** das partikuläre magnetische Material (8, 9, 10) in Material aufgenommen ist, das durch Härten gehärtet wurde, wie beispielsweise Klebstoff oder Farbe und mit dem Grundkörper (2) verbunden ist.

2. Katheter nach Anspruch 1, bei welchem das gehärtete Material in Form wenigstens eines Rings vorgesehen ist.

3. Katheter nach Anspruch 2, bei welchem der Katheter einen Endabschnitt umfaßt, der mit dem Grundkörper am distalen Ende verbunden ist, und wobei das gehärtete Material aus einem Haftmittel besteht, das zwischen dem Grundkörper und dem Endabschnitt aufgetragen ist.

4. Katheter nach Anspruch 1, bei welchem das gehärtete Material in Form wenigstens eines spiralförmigen Bandes vorgesehen ist.

5. Katheter nach Anspruch 1, bei welchem das das magnetische Material umfassende gehärtete Material aus einer Metallfarbe besteht, die auf der Außenoberfläche des Katheters aufgetragen ist.

6. Katheter nach Anspruch 5, bei welchem das magnetische Material schädlich für den Menschen ist und eine Beschichtung oben auf dem gehärteten Material aufgetragen ist, deren Material frei von magnetischem Material ist.

7. Katheter nach einem der vorstehenden Ansprüche, bei welchem das partikuläre magnetische Material aus ferromagnetischem Material besteht.

8. Katheter nach einem der vorstehenden Ansprüche, bei welchem das partikuläre magnetische Material aus stark paramagnetischem Material besteht der Gruppe, umfassend Kupfer, Mangan, Chrom, Nickel, Gadolinium und Dyprosium und Gemischen, Legierungen und Salzen aus diesen.

9. Verfahren zum Herstellen eines MR-sichtbaren Katheters durch Bereitstellen eines Katheters (1) und partikulären magnetischen Materials (8, 9, 10), **dadurch gekennzeichnet, daß** das partikuläre magnetische Material in dem durch Härten gehärteten Material aufgenommen ist wie beispielsweise Klebstoff oder Farbe, das mit dem Grundkörper (2) des Katheters (1) verbunden ist.

## Revendications

1. Cathéter (1) comprenant un matériau magnétique à particules (8, 9, 10), un corps de base en forme de tube (2) avec une extrémité distale et une extrémité proximale et au moins un lumen **caractérisé en ce que** le matériau magnétique à particules (8, 9, 10) a été reçu dans un matériau durci par durcissement, tel que de la colle ou de la peinture, relié au corps de base (2).

2. Cathéter selon la revendication 1, dans lequel le matériau durci est proposé sous la forme d'au moins un anneau.

3. Cathéter selon la revendication 2, dans lequel le cathéter comprend une section d'extrémité reliée au corps de base au niveau de l'extrémité distale, et dans lequel le matériau durci consiste en un moyen adhésif appliqué entre le corps de base et la section d'extrémité.

4. Cathéter selon la revendication 1, dans lequel le matériau durci est proposé sous la forme d'au moins une bande hélicoïdale.

5. Cathéter selon la revendication 1, dans lequel le matériau durci comprenant le matériau magnétique consiste en une peinture métallique appliquée sur la surface extérieure du cathéter.

6. Cathéter selon la revendication 5, dans lequel le matériau magnétique est nocif pour l'homme et un revêtement de matériau exempt de matériau magnétique a été appliqué sur le sommet du matériau durci.

7. Cathéter selon l'une des revendications précédentes, dans lequel le matériau magnétique à particules consiste en un matériau ferromagnétique.

8. Cathéter selon l'une quelconque des revendications précédentes, dans lequel le matériau magnétique à particules consiste en un matériau paramagnétique résistant du groupe composé de cuivre, manganèse, chrome, nickel, gadolinium et dysprosium et de mélanges, d'alliages et sels de ceux-ci.

9. Procédé de création d'un cathéter visible par résonance magnétique en proposant un cathéter (1) et un matériau magnétique à particules (8, 9, 10) **caractérisé en ce que** le matériau magnétique à particules a été reçu dans un matériau durci par durcissement, tel que de la colle ou de la peinture, relié au corps de base (2) du cathéter (1).
